# Europäisches Patentamt
## European Patent Office
## Office européen des brevets

(11) Publication number: **0 131 317**
**B1**

# EUROPEAN PATENT SPECIFICATION

(45) Date of publication of patent specification: **27.12.89**

(51) Int. Cl.⁴: **A 61 K 31/415**

(21) Application number: **84200525.8**

(22) Date of filing: **12.04.84**

(54) Use of Benzadac and of its salts for the manufacture of a medicament for the treatment of retinitis pigmentosa.

(30) Priority: **18.04.83 IT 2064983**

(43) Date of publication of application:
**16.01.85 Bulletin 85/03**

(45) Publication of the grant of the patent:
**27.12.89 Bulletin 89/52**

(84) Designated Contracting States:
**BE CH FR GB IT LI LU NL SE**

(56) References cited:
**BE-A- 891 914**
**GB-A-2 110 532**

**THE LANCET, no. 8276I, 10th April 1982, pages 849,850; M. TESTA et al.: "Pilot study of bendazac for treatment of cataract"**

**THE MERCK MANUAL OF DIAGNOSIS AND THERAPY, 14th edition, 1982, page 2005, edited by R. Berkow, published by Merck Sharp & Dohme Research Laboratories, Rahway, N.J., US;**

(73) Proprietor: **AZIENDE CHIMICHE RIUNITE ANGELINI FRANCESCO A.C.R.A.F. S.p.a.**
**Viale Amelia, 70**
**I-00181 Roma (IT)**

(72) Inventor: **Silvestrini, Bruno**
**Via Michelangelo Schipa, 15**
**I-00181 Roma (IT)**

(74) Representative: **Marchi, Massimo et al**
**c/o Marchi & Mittler s.r.l. Viale Lombardia 20**
**I-20131 Milano (IT)**

(58) References cited:
**MINERVA OFTALMOLOGICA, vol. 24, no. 4, 1982, pages 215-226; R. LEONI et al.: "Esperienze con Bendalina nella terapia della cataratta"**

**MINERVA OFTALMOLOGICA, vol. 24, no. 4, 1982, pages 227-233; G. BOSCHI et al.: "L'uso del bendazac lisina (Bendalina) nella terapia della cataratta"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# EP 0 131 317 B1

(56) References cited:

**INT. J. TISS. REAC., vol. 5, no. 2, April 1983, pages 217-225, Bioscience Ediprint Inc.; B. SILVESTRINI et al.: "Basic data supporting the use of the L-lysine salt of bendazac in cataract"**

**POLICLINICO, vol. 89, no. 6, 1982, pages 495-500; S. PERALTA et al.: "Saggio clinico-terapeutico della Bendalina nella cataratta"**

**Description**

This invention relates to the use of bendazac, ((1 - phenylmethyl - 1H - indazol - 3 - yl) - oxy) - acetic acid (Merck Index, IX ed.), and of the pharmaceutically acceptable acid salts thereof for the manufacture of a medicament for the treatment of retinitis pigmentosa.

Retinitis pigmentosa is a retinal degeneration characterized by:

night blindness;

progressive loss of peripheral vision, eventually leading to total blindness;

ophthalmoscopic changes consisting in dark mosaic like retinal pigmentation, attenuation of the retinal vessels, waxy pallor of the optic disc, and in the advanced forms, macular degeneration.

In some cases the pigmentation is lacking (Pearlman et al., Am. J. Ophthalmol. 81: (no. 4), 417—419 (1976)). Retinitis pigmentosa may be associated to degenerative opacity of the vitreous body and to cataract. A number of more complex syndromes are often associated to this disease, such as Usher's syndrome, responsible for deafness; Laurence-Moon syndrome, characterized by hypogonadism, mental retardation and obesity; Refsum's syndrome which may lead to mental retardation and dwarfism. Family history is prominent in retinitis pigmentosa; the pattern of inheritance may be autosomal recessive, autosomal dominant, or X-linked. The autosomal recessive form is the most common and may occur occasionally (McKusick, V. A. John Hopkins University Press, Baltimore, p. 963 (1978)). Disease incidence ranges from 1/2000 to 1/7000 according to the type of investigation and geographic location (Amman et al., J. Neurol, Sci. 2: 183—196 (1965); Merin and Auerbach, Surv. Ophthalmol. 20: 303—346 (1976)).

Retinitis pigmentosa was first described a century ago; nevertheless, its pathogenesis is still unkown despite the variety of hypotheses postulated as to its origin (Wirth, A., L'oculista Italiano No. 59: 52—55 (1982)). Not only is there no effective and safe treatment available for this disease at present, but the rational planning of pharmacological investigations is also very difficult; the lack of basic knowledge is, in fact, accompanied by the lack of animal models which suitably reproduce the corresponding human pathology, thereby enabling the laboratory study of potentially active substances. This explains why the problem of treating retinitis pigmentosa is generally based on working hypotheses confirmed by indirect experimental evidences. A typical example is the working hypothesis, prompted by the discovery of a local dopamine deficiency, which led to the study in man of a dopaminergic drug with encouraging results (Wirth, A., L'Oculista Italiano No. 59: 52—55 (1982).

We have now found that it is possible to treat retinitis pigmentosa in man by administering bendazac or a pharmaceutically acceptable salt thereof with an organic or inorganic base.

Bendazac is a known anti-inflammatory agent (Merck Index, IX ed.) and its lysine salt is known for oral treatment of cataract (Belgian Patent 891.914).

This invention is based on the original working hypothesis that retinitis pigmentosa is the result of a defect in the physiological mechanisms of protection, against the photo-oxidative processes involving free radicals; consequently, the free radicals which are continuously formed on the retina seem to cause, via a photo-oxidative process, a progressive damage to the structure. According to this hypothesis abnormal pigment deposition within the retina and the increased threshold in the perception of light stimuli would be, initially, nothing more than a secondary defensive process; biologically, these mechanisms seem just as important as the skin pigmentation following exposure to sun radiations. Retinal degeneration would be the result of a deficency in the protective physiological mechanisms and the secondary defensive processes mentioned above. On the basis of this original hypothesis, the search for drugs active on retinitis pigmentosa has two distinct objectives:

a) to normalize the physiological mechanisms of protection against the photo-oxidative processes involving free radicals. Unfortunately, this research approach is practically impossible due to the lack of the necessary basic knowledge;

b) to attenuate the biological effects of sun radiations on the retina.

Previous investigations conducted in our laboratories demonstrated that bendazac prevents protein denaturation produced by U.V. rays (Silvestrini et al., Inflammation, Biochemistry and Drug Interaction, A. Bertelli and J. C. Houck (eds.), Excerpta Medica Foundation, Amsterdam, pp. 283—288 (1969)); in view of the above hypothesis, it appeared potentially interesting in the treatment of retinitis pigmentosa as a drug capable of attenuating the biological effects of sun radiations on the retina. A recent observation has also shown that bendazac has a protective effect on photo-oxidative processes linked to free radicals in the photohemolysis test according to the method by Finazzi-Agrò et al., Experentia 35; 1445—1447 (1979). This test is based on the fact that protoporphyrin has, on some biological materials, a photosensitizing effect seemingly linked to the formation of free radicals (Lamola et al., Science 179: 1131 (1973); De Goeij et al., Clin. Chim. Acta 62: 287 (1975); Girotti, Biochem Biophys. Res. Commun. 72: 1367 (1976); Strom et al., Physiol. Chem. Phys. 9: 63 (1977)). In the above test bendazac showed a protective dose-related effect starting at concentrations around 3 µg/ml. A clinical study has been now performed with bendazac lysine salt in retinitis pigmentosa on a patient population of ten subjects with diagnostically confirmed advance retinitis pigmentosa. The patients were submitted to ophthalmological examination prior to the beginning of the study and then treated with bendazac lysine salt as dihydrate at an oral dose of 500 mg three times daily for a period of 6 months.

Ophthalmological tests were repeated at the following times: 1, 2, 4 and 6 months. The table summarizes the conditions of the patients before the treatment. Typical changes of the fundus included vessel restriction chorio-retinal dystrophy with pigmentation and optic nerve atrophy. In the ERG (electroretinogram), a typical change involved in retinitis pigmentosa was a curve with a decrease in the scoptopic component and monophasic wave of the cone function without photochromatic intervals. A checkup 1 month after the beginning of treatment showed an improvement in dark adaptation curve and ERG. Other 3 patients reported an improvement in visual acuity and dark adaptation which, however, were not confirmed by the tests performed. Two months after the beginning of treatment, 5 patients showed an improvement in the dark adaptation curve, ERG and visual acuity; 3 of these patients also reported a significant increase in visual field. Four and 6 months after the beginning of the treatment the results were unchanged. No severe drug-related side effect was observed necessitating the discontinuation of treatment.

TABLE

Conditions of the patients before the treatment with bendazac lysine salt in retinitis pigmentosa

| Case No. | Sex | Age | Characteristics of fundus | R | Visual field | L | Dark adaptation curve | E.R.G. |
|---|---|---|---|---|---|---|---|---|
| 1 | M | 65 | Typical changes | | Tubular | 15° | Characteristic | Flat |
| 2 | M | 28 | " " | | Tubular | 5° | " | " |
| 3 | M | 30 | " " | | Tubular | 15° | " | " |
| 4 | M | 50 | " " | | Tubular | 5° | " | " |
| 5 | M | 40 | " " | | Not detectable | | " | " |
| 6 | M | 50 | " " | 5° | Tubular | 10° | " | " |
| 7 | F | 52 | " " | | Tubular | 5° | " | " |
| 8 | F | 38 | " " | | ·Tubular | 10° | " | " |
| 9 | F | 32 | " " | | · Tubular | 5° | " | " |
| 10 | F | 61 | " " | | Not detectable | | " | " |

According to this invention there is provided the use of bendazac or a pharmaceutically acceptable salt thereof with an organic or inorganic base for the manufacture of a medicament for the treatment of retinitis pigmentosa in human beings. The preferred salt is bendazac lysinate.

The way of administration may be topic or systemic. A preferred embodiment of this invention provides the manufacture of an orally administratable medicament comprising a bendazac lysine salt, more particularly of the dihydrate lysine salt of bendazac, as an active agent against retenitis pigmentosa.

The dose can be set having regard to usual parameters such as the way of administration, the weight of the patient, the severity of the disease, the molar weight and the bioavailability of the administered compound.

Thus a human patient can receive a dose which causes a blood level of at least 10 γ/ml. Preferably the dose will be adjusted in such a way to give blood levels from 20 to 40 γ/ml.

In an adult human patient, a convenient daily dose which can be administered is from 600 to 1200 mg, preferably 900 mg, of bendazac or of the corresponding amount of a pharmaceutically acceptable salt thereof with an organic or inorganic base. This can be given in a single dose, but preferably it is given in two or three doses at equal intervals of time.

The course of treatment is continued indefinitely in view of the fact that bendazac exerts a protective effect and retinitis pigmentosa is a chronic, progressive disease. The preferred course of treatment contemplates the continuous oral administration of 900 mg a day of bendazac. In special circumstances, periods of treatment of 3—6 months with 600—1200 mg a day can be intercalated to periods of treatment of 2—4 months with a lower daily dose, such as 300 mg, of bendazac or of the corresponding amount of a pharmaceutically acceptable salt thereof with an organic or inorganic base.

The manufacture of a medicament according to this invention is normally performed in accordance with standard pharmaceutical practice.

Typical pharmaceutical compositions according to this invention include tablets, capsules and eye-drops. Preferably the composition is in unit dose form, for example a tablet or capsule, and includes from 500 to 1500 mg of bendazac lysine dihydrate salt together with one or more pharmaceutical carriers routinely used for preparing pharmaceutical compositions. Examples of such carriers include magnesium stearate, starch, lactose and sucrose.

Typical eye-drops composition contain from 0.1 to 1% of bendazac in a liquid pharmaceutical carrier or a mixture thereof.

According to this invention, a preferred pharmaceutical composition for oral use contains about 500 mg of bendazac lysine dihydrate salt and is administered three times a day.

## Claims

1. Use of bendazac and pharmaceutically acceptable salts thereof with organic or inorganic bases for the manufacture of a medicament for the treatment of retinitis pigmentosa in human beings.

2. Use of bendazac and pharmaceutically acceptable salts thereof according to claim 1 for the manufacture of an orally administratable medicament for the treatment of retinitis pigmentosa in human beings. ·

3. Use of bendazac and pharmaceutically acceptable salts thereof according to Claim 2, where the said medicament is in a dosage capable to impart blood levels in the range of 20—40 γ/ml.

## Patentansprüche

1. Verwendung von Bendazac und dessen pharmazeutisch verträglichen Salzen mit organischen oder anorganischen Basen zur Herstellung eines Arzneimittels zur Behandlung von Retinitis pigmentosa bei Menschen.

2. Verwendung von Bendazac und dessen pharmazeutisch verträglichen Salzen nach Anspruch 1, zur Herstellung eines oral verabreichbaren Arzneimittels zur Behandlung von Retinitis pigmentosa bei Menschen.

3. Verwendung von Bendazac und dessen pharmazeutisch verträglichen Salzen nach Anspruch 2, wobei das Arzneimittel in einer Dosierung vorliegt, die zu Blutspiegeln im Bereich von 20—40 γ/ml führt.

## Revendications

1. Utilisation du bendazac et des sels pharmaceutiquement acceptables de ce dernier avec des bases organiques ou inorganiques pour la fabrication d'un médicament pour le traitement de la rétinite pigmentaire chez l'homme.

2. Utilisation du bendazac et des sels pharmaceutiquement acceptables de ce dernier conformément à la revendication 1 pour la fabrication d'un médicament administrable par voie orale pour le traitement de la rétinite pigmentaire chez l'homme.

3. Utilisation du bendazac et des sels pharmaceutiquement acceptables de ce dernier selon la revendication 2, selon laquelle ledit médicament est sous la forme d'un dosage susceptible de donner des niveaux sanguins dans l'intervalle de 20—40 γ/ml.